# EUROPÄISCHE PATENTSCHRIFT

(11) **EP 0 832 668 B1**
(45) Veröffentlichungstag und Bekanntmachung des Hinweises auf die Patenterteilung: **15.05.2002**
(21) Anmeldenummer: 97250268.6
(22) Anmeldetag: 11.09.1997
(51) Int. Cl.: A61N 5/06

(54) **Bestrahlungsvorrichtung, insbesondere zur kosmetischen, diagnostischen und therapeutischen Lichtanwendung**
Illuminating device, in particular cosmetic, diagnostic-oriented and therapeutic use of light
Dispositif d'illumination plus particulièrement destiné à un emploi cosmétique, dans un but diagnostique et thérapeutique de la lumière

(30) Priorität: 26.09.1996 DE 19641216
(43) Veröffentlichungstag der Anmeldung: 01.04.1998
(73) Patentinhaber: DermaNova GmbH, 15745 Wildau (CH)
(72) Erfinder: Wilkens, Jan Henrik Dr. med., 66424 Homburg (DE)
(74) Vertreter: Effert, Bressel und Kollegen

(56) Entgegenhaltungen:
- EP-A- 0 406 454
- DE-A- 4 127 564
- US-A- 5 320 618
- US-A- 5 405 368

## Beschreibung

Die Erfindung betrifft eine Bestrahlungsvorrichtung, insbesondere zur kosmetischen, diagnostischen und therapeutischen Lichtanwendung, umfassend eine inkohärente Lichtquelle und einen die Lichtquelle umgebenden Reflektor, der in Abstrahlungsrichtung der Lichtquelle eine Öffnung aufweist, aus der die Strahlung austritt.

Die therapeutische Wirkung von Licht ist seit langem bekannt. Für die verschiedenen Krankheitsbilder sind unterschiedliche Vorrichtungen bekannt, die jeweils in ausgewählten, Spektralbereichen arbeiten.

Aus der DE 40 08 098 ist ein stabförmiger Beleuchtungskörper bekannt, der axial von einem Reflektor umgeben ist, der eine schlitzförmige Lichtaustrittsöffnung aufweist, die sich parallel zur Längsachse des Beleuchtungskörpers erstreckt und durch die aufeinander zulaufenden Schenkel des Reflektors gebildet ist, die an ihren Enden im Anschluß an die größte Annäherung der Schenkel mit einem Radius, der kleiner als der Krümmungsradius des Reflektors ist, entgegengesetzt zu dieser Krümmung umgebogen sind, wodurch eine erhöhte Strahlungsdichte erreicht wird.

Aus der DE-AS 17 64 685 ist eine elektrische Allzweck-Entladungslampe mit einem lichtdurchlässigen Lampenkolben, einem an eine Spannungsquelle anschließbaren Elektrodenpaar, einer ionisierbaren Füllung und einem auf der Kolbeninnenwand aufgebrachten Leuchtstoffbelag bekannt, mit der im Bereich oberhalb 290 nm Wellenlänge, insbesondere für Mittelultraviolettstrahlung eine Leistung von 6 bis 50 µW pro Lumen sichtbaren Lichts und für den Bereich des Nahultraviolett eine Leistung von 150 bis 700 µW pro Lumen sichtbaren Lichtes erzeugt werden soll, wobei die Leistung in den beiden Bereichen im Verhältnis von 1:8 bis etwa 1:40 gestaltet sein soll. Zusätzlich ist definiert, daß die Gesamtstrahlung pro Lumen etwa denselben Anteil haben soll, wie natürliches Tageslicht entsprechender Farbtemperatur. Abgesehen davon, daß der Leistungsvergleich Im/Watt irreführend ist und hier unsichtbares zu sichtbarem Licht ins Verhältnis gesetzt wird, errechnet sich, daß für den Gesamtbereich von 290 bis 320 nm eine maximale Leistung von 1,5 mW und für den Bereich von 320 bis 380 nm eine maximale Leistung von 42 mW emittiert werden soll. Ein Flächenbezug ist nicht angegeben.Weiterhin ist offenbart, daß derartige Lampen bis in den Bereich von 700 nm unterschiedliche Maxima z.B. bei 570 bis 595 oder 595 bis 625 nm haben können. Insgesamt wird in der Schrift auch Stand der Technik diskutiert, der sich mit Leuchtstofflampen spezieller Wellenlänge beschäftigt, die im Gegensatz zum Tageslicht ein scharfes Maximum bei verschiedenen Wellenlängen haben können. Dafür sind entsprechende Leuchtstoffmischungen angegeben, wobei charakteristischen Metallverbindungen für verschiedene Farbtemperaturen aufgelistet sind. Neben den erwähnten speziellen Verhältnissen im Bereich des UV-Lichtes sind für Lampen mit 40 W Nennleistung und einer Lichtleistung von 2100 bis 2300 Im die verschiedenen Leistungen natürlichen Lichtes gegenüber ausgewählten künstlichen Lampen dargestellt. Ziel der Erfindung ist es dort, eine Lampe vorzuschlagen, die während des 8 stündigen Arbeitstages eine unerwünschte Rötung der Haut (analog Sonnenbrand) vermeidet und trotzdem einen erwünschten Farbwiedergabeeffekt hat. Erwähnt sind auch verschiedene biologische Wirkungen in tabellarischer Form, insbesondere die Einwirkung von Licht auf den Augenfarbstoff, die Wirkung auf Zirbeldrüsen und Keimdrüsen durch Bestrahlung innerhalb einer Bereiches von 380 bis 700 nm Wellenlänge sowie die bakterientötende Wirkung von UV-Licht mit 254 nm Wellenlänge . Für den UV-Bereich ist insbesondere die Bildung von Vitaminen, die Inaktivierung von Mikroorganismen sowie die kosmetische Wirkung erwähnt. Hingewiesen wird ebenfalls darauf, daß UV-Strahlung Änderung in den Melaninmengen der Haut hervorrufen kann; dieser Effekt wird jedoch insbesondere der Einwirkung von Licht der Wellenlänge 290 bis 320 nm zugeschrieben und daher hervorgehoben, daß für diesem Bereich eine geringere Strahlung von den erfindungsgemäßen Allzwecklampen mit natürlichem Tageslichtspektrum vorzusehen sei.

Mit der DE 31 21 689 C 2 ist eine Leuchtstofflampe vorgeschlagen worden, die im Bereich von UVC und UVB durch den Glaskolben filternd wirkt. Für den Bereich von UVA ist angegeben, daß hier insbesondere ein Maximum oberhalb 350 nm mit einer Spektral-Breite von etwa 320 bis 400 nm gegeben sei und zusätzlich durch einen zweiten Leuchtstoff eine ausgeprägte Strahlungsemission im Oränge-Rot-Bereich bei etwa 650 nm vorgesehen ist. Dies soll bewirken, daß üblicherweise zur Bräunung oder zur Behandlung von Psoriasis eingesetzte Leuchtstofflampen in Solarien (DE-OS 26 28 091) keine Nebenerscheinungen wie Müdigkeit oder verminderte Aktivität erzeugen. Durch den Orange-Bereich soll eine einseitige Beeinflussung des Nerventonus und eine Gefäßweitstellung hervorgerufen werden, um Müdigkeit vorzubeugen. Allerdings ist angegeben, daß eine derartige Lampe im Bereich 404 und 437 nm (blau) ein für Quecksilber typisches Emissionsmaximum haben und daher es nicht möglich sei, den Blau-Bereich auszufiltern, was nach der Darstellung in der Beschreibung erwünscht ist.

In der DE-OS 34 31 692 wird vorgeschlagen, eine sonnenlichtähnliche Leuchtstofflampe mit fünf Energiemaxima bei etwa 320, 380, 450, 550, 650 nm zu verwenden, welche durch entsprechend dotierte Lampen mit den angegebenen Leuchtstoffen in einer Mischung erzielt werden sollen. Erwähnt ist, daß im UVA-Bereich Zellschäden reparabel sind und eine Regeneration der Augen ermöglicht wird, während im'Bereich unterhalb 320 nm die langwellige UVB-Strahlung die Bildung des Vitamin D 3 und eine Calziumresorption, Aktivierung des Stoffwechsels und Leistungssteigerung der Muskulatur und Kreislauforgane bewirke. Eine Sonnenbrandwirkung sei nur mit Wellenlängenbereichen unter 300 nm verbunden. Für den Bereich von 300 bis 400 nm werden etwa ähnliche Spektralemissionen angegeben, wie in der zuvor zitierten DE-AS 17 64 685.

In einer Vielzahl von Schriften sind für die unterschiedlichen Strahlungsbereiche, z.B. für rot bis dunkelrot (US-PS 32 87 586) oder für den blauen Bereich (DE-OS 19 22 416) geeignete Dotierungsmetalle angegeben. Spezielle Lampen (DD 201 207 und DD 221 374) wurden für therapeutische Fenster um 325 nm Wellenlänge konzipiert.Für die Behandlung von Hautstörungen ist in der DE-OS 32 39 417 eine Phosphor-Dotierung für eine optimierte Emission bei 340 - 400 nm vorgeschlagen worden.

In der DE 29 10 468 A 1 wird eine UV-Leuchtstoff-Strahlungsquelle für fotobiologische und fotochemische Zwecke, insbesondere zur Bräunungsbestrahlung vorgeschlagen, bei der der Kolben der Strahlungsquelle in Berührung mit einer Flüssigkeitschicht steht, vorzugsweise einer Wasserschicht oder einem Wasserbad. Ein solches Wasserbad kann auch als Wasserbett mit einer Liege ausgestaltet sein, wobei die Projektionsfläche, eine flexible transparente Abdeckung des Wasserbettes ist und die Wassertemperatur auf 30 bis 50°, vorzugsweise 35 bis 40° eingestellt werden soll. Dazu wird im Detail eine Anordnung der einzelnen Strahlungsquellen vorgeschlagen, die in rinnenförmigen Wasserbehältern, welche die Leuchtstoffröhren umschließen, angeordnet sind. Diese rinnenförmigen Elemente sollen mit Reflektoren auf der dem Bestrahlungsobjekt abgewandten Seite verse-. hen sein, um eine Lichtreflektion von dieser Kühleinrichtung in Richtung Projektionsfläche zu ermöglichen. Vorgeschlagen wird dabei die Verwendung von UV-Leuchtstofflampen im Spektrum von 300 bis 430 nm mit thermisch nicht hochbelastbaren Leuchtstoffen, gegebenenfalls sollen dem Kühlwasser Zusätze zur Beeinflussung des spektralen Transmissionsgrades und/oder der Herabsetzung der elektrischen Leitfähigkeit beigesetzt werden. Als erzielbare pigmentierungswirksame Strahlungsleistung, d.h. Bestrahlungsleistung im genannten UV-Bereich soll bei etwa 140 W/m² liegen. Fakultativ ist erwähnt, daß diese Bestrahlungseinrichtung auch für die medizinische Applikation (Diagnose und Therapie) geeignet ist, insbesondere auch für die Behandlung von Hautkrankheiten und Hautschäden.
Das Gerät hat sich nicht durchsetzen können, weil der Hauptzweck, die Bräunung, mit Solarien herkömmlicher Art billiger erreichbar ist und das Gerät in der medizinischen Praxis zu umständlich und zu teuer war. Ein Hauptgrund mag darin liegen, daß die Lampen sehr stark beansprucht wurden, da eine Oberflächentemperatur der Lampe von 35 bis 40° C insbesondere bei einer relativ hohen Nennleistung von 115 W die Lebensdauer der Lampen herabsetzt.

All diesen Vorrichtungen ist gemeinsam, daß die von Ihnen emittierte Strahlungsdichte zu gering ist. Dies führt entweder zu unvertretbar langen Behandlungszeiten oder aber gar zu keinem therapeutischen Effekt, da zum Teil der therapeutische Effekt erst ab einer bestimmten Strahlungsdichte einsetzt.

Ein weiterer Nachteil ist, daß es für bestimmte Spektralbereiche (z. B. Gelb) keine geeigneten Dotierstoffe gibt, mit denen entsprechende Strahlungsdichten erreichbar sind. Versucht man durch geeignete Filter eine breitbandige Lichtquelle ausgewählt in diesen Bereichen abstrahlen zu lassen, so wird die Strahlungsdichte durch die Filter weiter herabgesetzt.

Daher muß man bei Anwendungsgebieten mit erforderlichen hoher Strahlungsdichte weiterhin auf kostspielige und schwer handhabbare Laser zurückgreifen. insbesondere die Anwendung verschiedener Wellenlängen nacheinander bereitet mit Lasern große Schwierigkeiten.

Aus der US-PS 5, 405,368 ist eine gattungsgemäße Vorrichtung mit einer inkohärenten Lichtquelle bekannt, wobei die Lichtquelle durch einen Glaszylinder abgeschlossen ist und um die Lichtquelle ein Reflektor angeordnet ist. Der Reflektor hat im Querschnitt die Form einer Ellipse, wobei die Lichtquelle in einem Brennpunkt der Ellipse angeordnet ist. Auf der der Lichtquelle abgewandten Seite weist der Reflektor eine Öffnung auf. Vor der Öffnung ist ein Satz von optischen Filtern und eine Irisblende angeordnet. Als Lichtquelle wird zum Beispiel eine Blitzleuchte vorgeschlagen. Wird nun die Vorrichtung auf die zu behandelnde Körperpartie aufgesetzt, so liegt diese Partie im zweiten Brennpunkt der Ellipse. Alternativ wird vorgeschlagen, den Reflektor parabolisch und rund im Querschnitt auszubilden. Die Irisblende kontrolliert die Länge und Breite der bestrahlten Fläche, wobei die maximale Länge durch die Länge der Lichtquelle begrenzt ist. Bei einer vorgeschlagenen Leuchtenlänge von 8 cm werden allerdings nur 5 cm in der Mitte der Leuchte ausgenutzt, wo eine annähernd gleichförmige Abstrahlung stattfindet. Durch Schließen der Irisblende kann die Länge der bestrahlten Fläche auf eine Länge von 1 mm verkürzt werden, ebenso wie die Breite. Bei einer Breite von b= 5 mm kann die Breite durch Schließen der Irisblende bis zu 1 mm verkürzt werden. Dies entspricht einer maximalen Öffnungsbreite d von einem Fünftel des Durchmessers. Damit sind Energiedichten im Bereich von 30 bis 100 J/cm² erreichbar. Bei Verwendung eines optischen Bandpasses im Bereich von 500 bis 650 nm reduziert sich die Energiedichte um 80%, so daß auf der Haut noch Energiedichten von 6 bis 20 J/cm² erreicht werden.

Nachteilig an der bekannten Vorrichtung ist, daß aufgrund der Energiedichten die Lebensdauer der optischen Filter sehr kurz ist bzw. die einschlägigen Hersteller eine Garantie für die Funktionsfähigkeit ablehnen. Andererseits sind die mit der Vorrichtung maximal erzielbaren Energiedichten für die Anwendungsgebiete "Verödung von Venen" und "Zerstörung von Tätowierungspigmenten" nicht ausreichend groß genug.

Der Erfindung liegt daher das technische Problem zugrunde, eine Bestrahlungsvorrichtung, insbesondere zur kosmetischen, diagnostischen und therapeutischen Lichtanwendung, zu schaffen, mit der unter Einsatz einer inkohärenten Lichtquelle ausreichende Strahlungsdichten bei langer Lebensdauer der Einzelelemente erzielbar sind.

Die Lösung des Problems ergibt sich aus den Merkmalen des Patentanspruches 1. Anspruch 1 betrifft eine Bestrahlungsvorrichtung, insbesondere zur kosmetischen, diagnostischen und therapeutischen Lichtanwendung, umfassend eine inkohärente Lichtquelle und einen die Lichtquelle umgebenden Reflektor, der in Abstrahlrichtung einer Spalt aufweist. Durch die Anordnung eines Spektralzerlegeelmentes im aus dem Spalt austretenden Strahlengang der Lichtquelle, in dessen Ausgangsstrahlengang ein faseroptischer Querschnittswandler derart angeordnet ist, daß dessen kreisförmiges Ende auf der dem Spektralzerlegeelment abgewandten Seite sich befindet, wobei faseroptischer Querschnittswandler und Spektralzerlegeelment zueinander relativ bewegbar sind, kann nahezu verlustfrei ein ausgewählter spektraler Bereich der Lichtquelle in den faseroptischen Querschnittswandler eingekoppelt werden. Weitere vorteilhafte Ausgestaltungen der Erfindung ergeben sich aus den Unteransprüchen.

In einer weiteren Ausführungsform ist in Abstrahlrichtung der Lichtquelle aus dem Spalt ein zylindrischer Ellipsenreflektor angeordnet, in dessen dem Scheitel zugewandten Brennpunkt die Lichtquelle angeordnet ist. Im zweiten Brennpunkt des zylindrischen Ellipsenreflektors ist das Spektralzerlegeelement angeordnet, wobei das Spektralzerlegeelement auch dreh- oder schwenkbar ausgebildet sein kann. Der im Ausgangsstrahlengang des Spektralzerlegeelementes angeordnete faseroptischer Querschnittswandler kann ebenfalls in Vertikalund/oder Horizontalrichtung verschiebbar und/oder dreh- und/oder schwenkbar ausgebildet sein. In einer weiteren Ausführungsform wird an Stelle des zylindrischen Ellipsenreflektors ein zylindrischer Doppelparabel-Reflektor verwendet.

Der die Lichtquelle umgebende Reflektor kann auch durch einen Ellipsoid-Reflektor mit zusätzlichem Hohlspiegel gebildet werden. Dazu wird die Lichtquelle in einem dem Scheitel des zylindrischen Ellipsenreflektors zugewandten Brennpunkt angeordnet und der Hohlspiegel mit dem Spalt wird in dem anderen Brennpunkt des zylindrischen Ellipsenreflektors angeordnet. Die Ellipse wird durch ein Kreissegment mit dem ersten Brennpunkt als Mittelpunkt abgeschlossen. Dadurch werden im Prinzip alle Strahlen im Spalt vereinigt. Durch die variable Einstellung der Breite des Spaltes des Reflektors kann je nach Anwendungsgebiet die Strahlungsdichte oder die abgegebene Gesamtstrahlung angepaßt werden. Das Spektralzerlegeelement kann in allen Ausführungsformen z. B. als Gitter oder Prisma ausgebildet sein. Weiter kann der Vorrichtung ein Kühlmittelaggregat zugeordnet sein. Das Kühlmittelaggregat kann in festen, programmierbaren Zeitintervallen die bestrahlte Hautpartie mit Kühlmittel besprühen oder aber über einen an der Hautpartie angeordneten Temperaturoder Infrarotsensor in Wirkverbindung stehen, so daß bei überschreiten einer einstellbaren Grenztemperatur die Hautpartie mit Kühlmittel besprüht wird. Der Siedepunkt der Kühlmittel kann dabei zwischen -80° und -10° C liegen. Als Kühlmittel kann z.B. Freon Verwendung finden. Als Lichtquelle kommen z. B. aus anderen Anwendungsgebieten bekannte Hoch- oder Niederdruckgasentladungsleuchten oder Glühleuchten wie z.B. Halogenleuchten in Betracht. Zusätzlich können die Leuchten mit einer Leuchtstoffschicht beschicht sein.

Die Erfindung wird nachfolgend anhand von bevorzugten Ausführungsbeispielen näher erläutert. Die Figuren zeigen:
- Fig. 1:: einen Querschnitt durch die Vorrichtung mit Lichtquelle und umgebenden Reflektor,
- Fig.2:: eine schematische Perspektivdarstellung einer Vorrichtung mit einem umgebenden Reflektor und weiteren Bauteilen,
- Fig.3:: einen Querschnitt durch eine weitere Vorrichtung mit ei nem umgebenden Reflektor und einem Zusatzreflektor,
- Fig 4:: einen Querschnitt durch eine weitere Vorrichtung mit Licht quelle und einem die Lichtquelle umgebenden Reflektor, der durch einen zylindrischen Ellipsenreflektor mit einem Hohlspiegel gebildet ist,
- Fig. 5:: ein Diagramm der abgestrahlten Gesamtleistung in % über dem Verhältnis Spaltbreite/Umfang und
- Fig. 6:: ein Diagramm der Verstärkung der Leistungsdichte über dem Verhältnis Spaltbreite/Umfang.

Die Bestrahlungsvorrichtung 1 umfaßt eine mit Gasmolekülen 2 gefüllte Lichtquelle 3, die durch einen Glaszylinder 4 umschlossen ist. Um den Glaszylinder 4 ist anliegend oder beabstandet ein zylinderförmiger Reflektor 5 angeordnet, der einen Spalt 6 mit der Breite d aufweist, der sich in Längsrichtung des Reflektors 5 erstreckt. Als Lichtquelle 3 wird zum Beispiel eine bekannte Xenon-Entladungslampe mit 300 mm Länge und einem Durchmesser von 10 mm verwendet. Der die Lichtquelle 3 umgebende Reflektor 5 kann dabei über die volle Länge oder aber auch nur teilweise geschlitzt ausgeführt sein. Die Breite d des Spaltes 6 ist kleiner als U/(4 x π) des Reflektors 5, wobei U der Umfang des geschlossenen Reflektors 5 ist. Die Leuchtstoffröhre erzeugt eine diffuse Strahlung 7 in einem weiten Spektralbereich. Ein geringer Anteil der Strahlung 7 tritt direkt durch den Spalt 6 aus, wohingegen der größte Teil der diffusen Strahlung 7 auf den Reflektor 5 trifft. Dort wird die Strahlung 7 reflektiert, wobei ein geringer Anteil von ca. 1% bei jedem Reflexionsvorgang absorbiert wird. Die Strahlung 7 wird solange am Reflektor 5 reflektiert, bis diese auf den Spalt 6 fällt und austreten kann, so daß der Spalt 6 die Abstrahlungsrichtung der Lichtquelle 3 definiert.

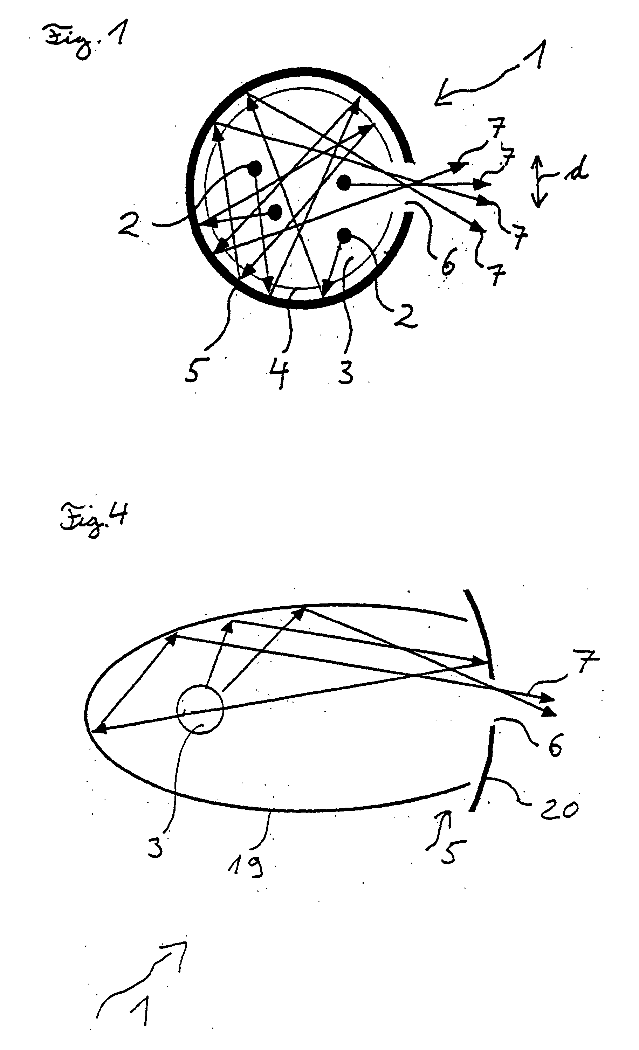

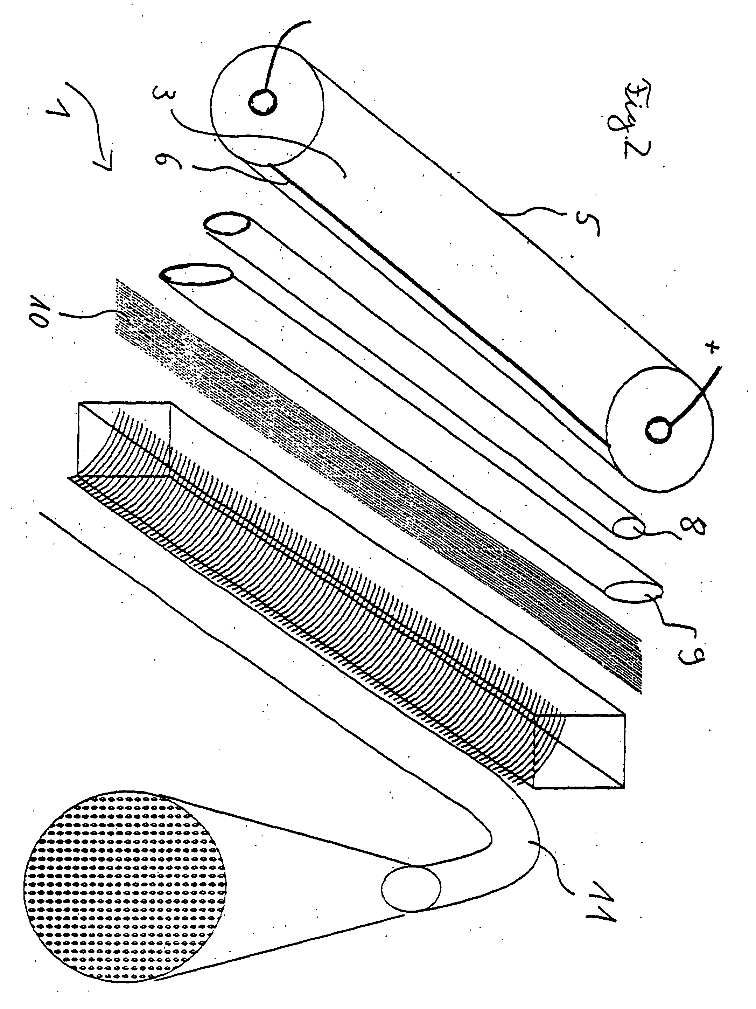

In der Fig.2 ist eine schematische Perspektivansicht der Bestrahlungsvorrichtung 1 dargestellt. Die Bestrahlungsvorrichtung 1 umfaßt die Lichtquelle 3 mit dem die Lichtquelle 3 umgebenden Reflektor 5 mit Spalt 6. In Abstrahlrichtung der Lichtquelle 3 ist ein Kondensor 8 mit kleiner Brennweite angeordnet, in dessen Brennebene ein weiterer Kondensor 9 mit großer Brennweite angeordnet ist. In der Brennebene des Kondensors 9 ist ein Spektralzerlegeelement 10 angeordnet. Das Spektralzerlegeelement 10 ist zum Beispiel als Gitter oder Prisma ausgebildet. Im Ausgangsstrahlengang des Spektralzerlegeelementes 10 ist ein faseroptischer Querschnittswandler 11 angeordnet. Der faseroptische Querschnittswandler 11 hat auf der dem Spektralzerlegeelement 10 zugewandten Seite z.B. die Abmessungen 300 mm X 0,3 mm und auf der dem Spektralzerlegeelement 10 abgewandten Seite einen kreisrunden Querschnitt von ca. 1 cm Durchmesser. Die aus dem Spalt 6 des Reflektors 5 austretende Strahlung trifft auf den Kondensor 8 und wird von diesem auf den Kondensor 9 abgebildet. Der Kondensor 8 wirkt zusammen mit dem Kondensor 9 dabei als Kollimator. Der Kondensor 9 bildet die Strahlung auf das Spektralzerlegeelement 10 ab, wo die Strahlung spektral zerlegt wird. Das Spektralzerlegeelement 10 und/oder der faseroptische Querschnittswandler 11 sind verschieb- und/oder verdrehbar ausgebildet, so daß diese zueinander so justiert werden können, daß die spektral zerlegte Strahlung auf den Eingang des faseroptischen Querschnittswandler 11 abgebildet wird. Die aus dem kreisrunden Querschnitt des faseroptischen Querschnittwandlers 11 austretende Strahlung kann dann direkt zur Bestrahlung eines Patienten benutzt werden. Durch Verschiebung des Spektralzerlegeelementes 10 oder des faseroptischen Querschnittwandlers 11 läßt sich eine feine spektrale Abtastung realisieren, indem nur ausgewählte Frequenzbereiche in den faseroptischen Querschnittswandler 11 eingekoppelt werden.

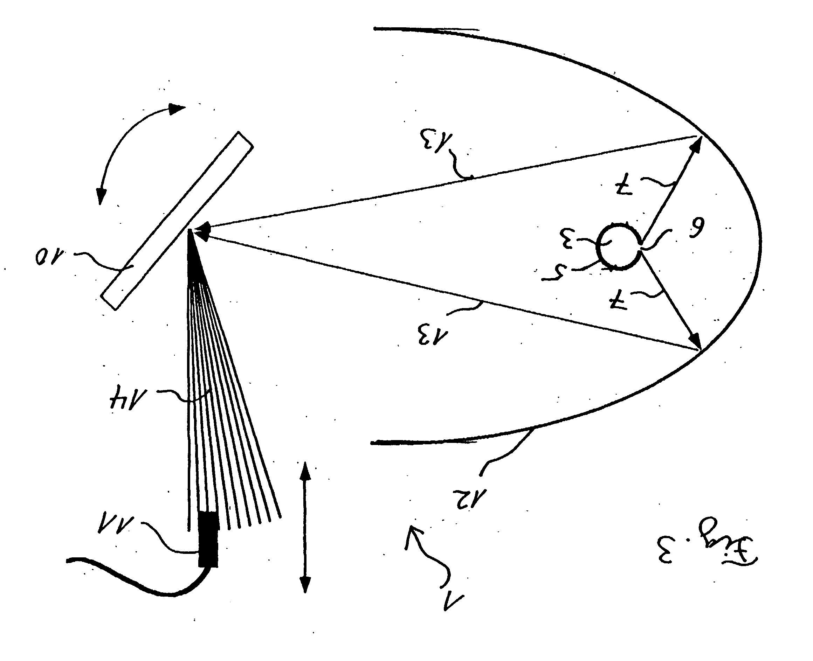

In der Fig.3 ist eine Vorrichtung 1 mit Doppelreflektorstruktur dargestellt. In Abstrahlrichtung der Lichtquelle 3 aus dem Spalt 6 des Reflektors 5 ist ein zylindrischer Ellipsenreflektor 12 angeordnet, wobei die Lichtquelle 3 in dem dem Scheitel des zylindrischen Ellipsenreflektors 12 zugewandtem Brennpunkt angeordnet ist. Im anderen Brennpunkt des zylindrischen Ellipsenreflektors 12 ist das Spektralzerlegeelement 10 angeordnet, das verdrehbar ausgebildet ist. Im Ausgangsstrahlengang des Spektralzerlegeelements 10 ist der faseroptische Querschnittswandler 11 angeordnet, der verschiebbar ausgebildet ist. Neben der dargestellten vertikalen Verschiebung kann der faseroptische Querschnittswandler 11 bei Bedarf auch horizontal verschiebbar ausgebildet sein. Die von der Lichtquelle 3 durch den Spalt 6 des Reflektors 5 austretende Strahlung 7 trifft auf die Innenwand des zylindrischen Ellipsenreflektors 12. Der zylindrische Ellipsenreflektor 12 reflektiert die Strahlung 7 in den zweiten Brennpunkt des zylindrischen Ellipsenreflektors 12. Die reflektierte Strahlung 13 trifft im zweiten Brennpunkt auf das Spektralzerlegeelement 10 und wird von diesem spektral aufgespalten. Das Spektralzerlegeelement 10 ist in dieser Ausführungsart vorzugsweise als Gitter ausgebildet. Im Strahlengang der Gitterstrahlung 14 ist der faseroptische Querschnittswandler 11 angeordnet, in den der jeweilige spektrale Anteil der Gitterstrahlung 14 nach Lage vom Spektralzerlegeelement 10 zu dem faseroptischen Querschnittswandler 11 eingekoppelt wird. Durch Verschiebung des faseroptischen Querschnittswandlers 11 in Horizontalrichtung kann ein bestimmter Spektralbereich ausgewählt werden, ebenso wie durch Drehung des Spektralzerlegeelementes 10. Durch die vertikale Verschiebung des faseroptischen Querschnittswandlers 11 kann die Bandbreite des eingekoppelten Spektralbereiches variiert werden, so daß bei Bedarf das gesamte Spektrum eingekoppelt werden kann.

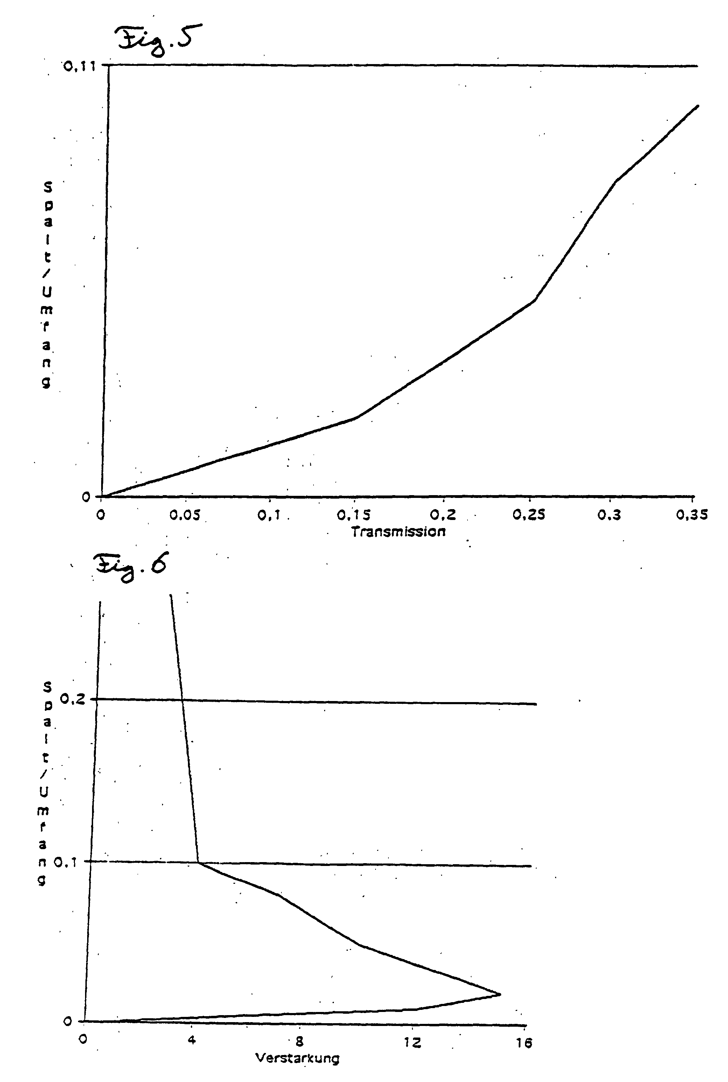

In der Fig.4 ist eine weitere mögliche Ausführungsform der Bestrahlungsvorrichtung 1 dargestellt. Der die Lichtquelle 3 umgebende Reflektor 5 wird dabei durch einen zylindrischen Ellipsenreflektor 19 und einen Hohlspiegel 20 gebildet. Die Lichtquelle 3 ist in einem dem Scheitel des zylindrischen Ellipsenreflektors 19 zugewandten Brennpunkt angeordnet. In dem anderen Brennpunkt des zylindrischen Ellipsenreflektors 19 ist der Hohlspiegel 20 angeordnet, der einen Spalt 6 aufweist. Aufgrund der Eigenschaften der Ellipse, nämlich der Abbildung von einem Brennpunkt in den anderen Brennpunkt, tritt ein größer Anteil der von der Lichtquelle 3 emittierten Strahlung 7 ohne vorherige Reflexion direkt aus den Spalt 6 aus, so daß sich die erreichbare Strahlungsleistungsdichte weiter erhöht.

Bei Verwendung einer 300 mm Blitzlampe mit einer Blitzenergie von 8000 J ergibt sich eine Blitzleistungsdichte von 90 J/cm². Durch die Ausbildung des Spaltes 5 mit einer Breite d kleiner als U/(4 x π) läßt sich die Leistungsdichte auf über 1000 J/cm² erhöhen, so daß bei Annahme von 50% Einkopplungsverlusten eine effektive Energiedichte von über 500 J/cm² erreicht wird. Zerlegt man das von ca. 200-1200 nm sich erstreckende Spektrum in fünf gleichgroße Wellenlängenbereiche, so ergibt sich immer noch eine effektive Energiedichte von ca. 100 J/cm² pro Wellenlängenbereich. Dies übertrifft sogar derzeitige Feststofflaser, deren dünner Strahl mit Hilfe eines X-Y-Scanners erst künstlich aufgeweitet werden muß, um Hautverletzungen zu vermeiden.

Neben den bereits genannten Anwendungsgebieten ist auch die Verwendung in Endoskopen möglich. So ist zum Beispiel eine sehr helle Ausleuchtung im blauen UV-Bereich möglich, wo über eine 1 mm-Faser 1000 mW Lichtleistung appliziert werden kann, was einer Lichtleistung von ca. 1000 blauen LED's entspricht. In Folge dieser hellen Ausleuchtung ist es ebenfalls möglich, eine steady state Fluoreszenzdetektion mit Chip-Endoskopen ohne Bildverstärkung durchzuführen. Ebenso können in der photodynamischen Therapie im roten Spektralbereich um 635 nm bisher verwendete Feststofflaser ersetzt werden.

Aus dem Diagramm der Fig. 5 ist zu entnehmen, daß die abgestrahlte Gesamtleistung in % durch Verwendung eines Spaltes 6 mit Verringerung der Breite d monoton abnimmt, jedoch die Leistungsdichte aufgrund der Verstärkung, wie aus Fig. 6 zu entnehmen ist, monoton bis zum Erreichen eines absoluten Maximums zunimmt. Der anschließende Abfall der Verstärkung bei sehr kleinen Spaltbreiten ist auf die überproportionale Zunahme der Reflexionen zurückzuführen. Die in den Figuren dargestellten Kurven sind an einer zylindrischen Lichtquelle mit einem diese umgebenden zylindrischen Reflektor 5 gemessen worden.

### Bezugszeichenliste

- 1): Bestrahlungsvorrichtung
- 2): Gasmolekül
- 3): Lichtquelle
- 4): Glaszylinder
- 5): Reflektor
- 6): Spalt
- 7): Strahlung
- 8): Kondensor
- 9): Kondensor
- 10): Spektralzerlegeelement
- 11): faseroptischer Querschnittswandler
- 12): zylindrischer Ellipsenreflektor
- 13): reflektierte Strahlung
- 14): Gitterstrahlung
- 19): zylindrischer Ellipsenreflektor
- 20): Hohlspiegel

## Patentansprüche

1. Bestrahlungsvorrichtung, insbesondere zur kosmetischen, diagnostischen und therapeutischen Lichtanwendung, umfassend eine inkohärente Lichtquelle und einen die Lichtquelle umgebenden Reflektor, der in Abstrahlrichtung einen Spalt aufweist,
**dadurch gekennzeichnet, daß**
im aus dem Spalt austretenden Strahlengang der Lichtquelle (3) ein Spektralzerlegeelement (10) und in dessen Ausgangsstrahlengang ein faseroptischer Querschnittswandler (11) derart angeordnet ist, daß dessen kreisförmiges Ende auf der dem Spektralzerlegeelement (10) abgewandten Seite sich befindet, wobei faseroptischer Querschnittswandler (11) und Spektralzerlegeelement (10) zueinander relativ bewegbar sind, derart, daß ein ausgewählter Frequenzbereich der Lichtquelle (3) im den faseroptischen Querschnittswandler (11) eingekoppelt wird.

2. Vorrichtung nach Anspruch 1, **dadurch gekennzeichnet, daß** in Abstrahlrichtung der Lichtquelle (3) aus dem Spalt (6) ein zylindrischer Ellipsenreflektor (12) angeordnet ist, in dessen dem Scheitel zugewandten Brennpunkt die Lichtquelle (3) und im zweiten Brennpunkt das Spektralzerlegeelement (10) angeordnet ist.

3. Vorrichtung nach Anspruch 1, **dadurch gekennzeichnet, daß** in Abstrahlrichtung der Lichtquelle (3) aus dem Spalt (6) ein zylindrischer Doppelparabel-Reflektor angeordnet ist und in der der Abstrahlrichtung der Lichtquelle (3) entgegengesetzten Richtung das Spektralzerlegeelement (10) angeordnet ist.

4. Vorrichtung nach Anspruch 1, **dadurch gekennzeichnet, daß** der Reflektor (5) durch einen zylindrischen Ellipsenreflektor (19) und einen Hohlspiegel (20) gebildet ist, wobei der Hohlspiegel (20) den Spalt (6) aufweist, die Lichtquelle (3) in einem dem Scheitel des zylindrischen Ellipsenreflektors (19) zugewandten Brennpunkt angeordnet ist und der Hohlspiegel (20) in einem zweiten, vom Scheitel des zylindrischen Ellipsenreflektors (19) abgewandten Brennpunkt des zylindrischen Ellipsenreflektors (19) angeordnet ist.

5. Vorrichtung nach einem der Ansprüche 1 bis 4, **dadurch gekennzeichnet, daß** die Breite d des Spalts variabel einstellbar ist.

6. Vorrichtung nach einem der Ansprüche 1 bis 5, **dadurch gekennzeichnet, daß** die Lichtquelle (3) wahlweise gepulst oder kontinuierlich ansteuerbar ist.

7. Vorrichtung nach einem der vorangegangenen Ansprüche, **dadurch gekennzeichnet, daß** die Lichtquelle (3) relativ zum Reflektor (5) verschiebbar ausgebildet ist und/oder exzentrisch zum Reflektor (5) angeordnet ist.

8. Vorrichtung nach einem der vorangegangenen Ansprüche, **dadurch gekennzeichnet, daß** der Reflektor (5) als Metallkörper mit verspiegelter Innenfläche ausgebildet ist.

9. Vorrichtung nach einem der vorangegangenen Ansprüche, **dadurch gekennzeichnet, daß** das Spektralzerlegeelement (10) dreh- oder schwenkbar und/oder der faseroptische Querschnittswandler (11) verschiebbar und/oder dreh- und schwenkbar ausgebildet ist.

10. Vorrichtung nach einem der vorangegangenen Ansprüche, **dadurch gekennzeichnet, daß** zwischen der Lichtquelle (3) und dem Spektralzerlegeelement (10) ein Kollimator angeordnet ist.

11. Vorrichtung nach Anspruch 10, **dadurch gekennzeichnet, daß** der Kollimator eingangsseitig durch einen ersten Kondensor (8) mit kleiner Brennweite und ausgangsseitig durch einen zweiten Kondensor (9) mit großer Brennweite gebildet ist, wobei der zweite Kondensor (9) in der Brennebene des ersten Kondensors (8) angeordnet ist.

12. Vorrichtung nach einem der vorangegangenen Ansprüche, **dadurch gekennzeichnet, daß** das Spektralzerlegeelement (10) als Prisma oder Gitter ausgebildet ist.

13. Vorrichtung nach einem der vorangegangenen Ansprüche, **dadurch gekennzeichnet, daß** der Vorrichtung (1) ein Kühlmittelaggregat zugeordnet ist, das in festen, programmierbaren Zeitintervallen die bestrahlte Hautpartie mit Kühlmittel versprüht.

14. Vorrichtung nach einem der Ansprüche 1 bis 12, **dadurch gekennzeichnet, daß** der Vorrichtung (1) ein Kühlmittelaggregat zugeordnet ist, das mit einem Temperatur- oder Infrarotsensor in Wirkverbindung steht und bei überschreiten einer einstellbaren Grenztemperatur Kühlmittel versprüht.

15. Vorrichtung nach Anspruch 13 oder 14, **dadurch gekennzeichnet, daß** der Siedepunkt des Kühlmittels zwischen -80° bis -10°C liegt.

16. Vorrichtung nach einem der Ansprüche 1 bis 15, **dadurch gekennzeichnet, daß** die Lichtquelle (3) als Hoch- oder Niederdruckgasentladungsleuchte oder als Glühleuchte, insbesondere als Halogenleuchte ausgebildet ist.

17. Vorrichtung nach einem der vorangegangenen Ansprüche, **dadurch gekennzeichnet, daß** die Lichtquelle (3) am Glaskörper mit einer Leuchtstoffschicht beschichtet ist.

## Claims

1. Illuminating apparatus, more especially for the cosmetic, diagnostic and therapeutic use of light, including an incoherent light source and a reflector, which surrounds the light source and has a gap in the reflected direction, **characterised in that** a spectral scanning element (10) is so disposed in the beam path of the light source (3) emerging from the gap, and a fibre-optic cross-sectional converter (11) is so disposed in the output beam path of said element that the circular end of said converter that the circular end of said converter is situated on the side remote from the spectral scanning element (10), the fibre-optic cross-sectional converter (11) and the spectral scanning element (10) being displaceable relative to each other in such a manner that a selected frequency range of the light source (3) is introduced into the fibre-optic cross-sectional converter (11).

2. Apparatus according to claim 1, **characterised in that** a cylindrical ellipse reflector (12) is disposed in the reflected direction of the light source (3) from the gap (6), the light source (3) being disposed at the focal point facing the crown, and the spectral scanning element (10) being disposed at the second focal point.

3. Apparatus according to claim 1, **characterised in that** a cylindrical double-parabola reflector is disposed in the reflected direction of the light source (3) from the gap (6), and the spectral scanning element (10) is disposed in the direction opposite the reflected direction of the light source (3).

4. Apparatus according to claim 1, **characterised in that** the reflector (5) is formed by a cylindrical ellipse reflector (19) and a concave mirror (20), the concave mirror (20) having the gap (6), the light source (3) being disposed at a focal point facing the crown of the cylindrical ellipse reflector (19), and the concave mirror (20) being disposed at a second focal point of the cylindrical ellipse reflector (19) remote from the crown of the cylindrical ellipse reflector (19).

5. Apparatus according to one of claims 1 to 4, **characterised in that** the width d of the gap can be variably set.

6. Apparatus according to one of claims 1 to 5, **characterised in that** the light source (3) is selectively pulsed or continuously actuatable.

7. Apparatus according to one of the preceding claims, **characterised in that** the light source (3) is adapted to be displaceable relative to the reflector (5) and/or is disposed eccentrically relative to the reflector (5).

8. Apparatus according to one of the preceding claims, **characterised in that** the reflector (5) is in the form of a metal body having a mirror-coated internal surface.

9. Apparatus according to one of the preceding claims, **characterised in that** the spectral scanning element (10) is adapted to be rotatable or pivotable and/or the fibre-optic cross-sectional converter (11) is adapted to be displaceable and/or rotatable and pivotable.

10. Apparatus according to one of the preceding claims, **characterised in that** a collimator is disposed between the light source (3) and the spectral scanning element (10).

11. Apparatus according to claim 10, **characterised in that** the collimator is formed at the input side by a first condenser (8) having a short focal length and at the output side by a second condenser (9) having a long focal length, the second condenser (9) being disposed in the focal plane of the first condenser (8).

12. Apparatus according to one of the preceding claims, **characterised in that** the spectral scanning element (10) is in the form of a prism or lattice.

13. Apparatus according to one of the preceding claims, **characterised in that** the apparatus (1) has associated therewith a coolant unit which sprays the illuminated main portion with coolant at fixed, programmable intervals of time.

14. Apparatus according to one of claims 1 to 12, **characterised in that** the apparatus (1) has associated therewith a coolant unit which is in operative connection with a temperature or infrared sensor and sprays coolant when an adjustable limit temperature is exceeded.

15. Apparatus according to claim 13 or 14, **characterised in that** the boiling point of the coolant is between -80° and -10° C.

16. Apparatus according to one of claims 1 to 15, **characterised in that** the light source (3) is in the form of a high- or low-pressure gas discharge light or in the form of an incandescent light, more especially in the form of a halogen light.

17. Apparatus according to one of the preceding claims, **characterised in that** the light source (3) is coated with a layer of fluorescent material on the glass body.

## Revendications

1. Dispositif d'irradiation, en particulier pour une utilisation cosmétique, diagnostique et thérapeutique de la lumière, comprenant une source de lumière incohérente et un réflecteur entourant la source de lumière qui présente une fente dans la direction de rayonnement, **caractérisé en ce que** sur le chemin optique de la source de lumière (3) sortant de la fente est disposé un élément d'analyse spectrale (10), et sur le chemin optique sortant de celui-ci est disposé un convertisseur de section à fibre optique (11) de telle sorte que l'extrémité circulaire de celui-ci se trouve du côté détourné de l'élément d'analyse spectrale (10), le convertisseur de section à fibre optique (11) et l'élément d'analyse spectrale (10) étant mobiles l'un par rapport à l'autre, de telle sorte qu'une plage de fréquences sélectionnée de la source de lumière (3) est modulée dans le convertisseur de section à fibre optique (11).

2. Dispositif selon la revendication 1, **caractérisé en ce que** dans la direction de rayonnement de la source de lumière (3) sortant de la fente (6) est disposé un réflecteur elliptique cylindrique (12), dans le foyer de celui-ci, qui est orienté vers le sommet, est disposée la source de lumière (3) et dans le deuxième foyer est disposé l'élément d'analyse spectrale (10).

3. Dispositif selon la revendication 1, **caractérisé en ce que** dans la direction de rayonnement de la source de lumière (3) sortant de la fente (6) est disposé un réflecteur cylindrique à double parabole et dans la direction opposée à la direction de rayonnement de la source de lumière (3) est disposé l'élément d'analyse spectrale (10).

4. Dispositif selon la revendication 1, **caractérisé en ce que** le réflecteur (5) se compose d'un réflecteur elliptique cylindrique (19) et d'un miroir concave (20), le miroir concave (20) présentant la fente (6), la source de lumière (3) étant disposée dans un foyer orienté vers le sommet du réflecteur elliptique cylindrique (19) et le miroir concave (20) étant disposé dans un deuxième foyer du réflecteur elliptique cylindrique (19) détourné du sommet du réflecteur elliptique cylindrique (19).

5. Dispositif selon l'une quelconque des revendications 1 à 4, **caractérisé en ce que** la largeur d de la fente est réglable de façon variable.

6. Dispositif selon l'une quelconque des revendications 1 à 5, **caractérisé en ce que** la source de lumière (3) peut être commandée au choix en régime pulsé ou en continu.

7. Dispositif selon l'une quelconque des revendications précédentes, **caractérisé en ce que** la source de lumière (3) est réalisée de façon mobile par rapport au réflecteur (5) et/ou est disposée de façon excentrique par rapport au réflecteur (5).

8. Dispositif selon l'une quelconque des revendications précédentes, **caractérisé en ce que** le réflecteur (5) est réalisé sous la forme d'un corps métallique avec une surface intérieure métallisée.

9. Dispositif selon l'une quelconque des revendications précédentes, **caractérisé en ce que** l'élément d'analyse spectrale (10) est réalisé de façon orientable ou pivotante et/ou le convertisseur de section à fibre optique (11) est réalisé de façon mobile et/ou de façon orientable et pivotante.

10. Dispositif selon l'une quelconque des revendications précédentes, **caractérisé en ce qu'**entre la source de lumière (3) et l'élément d'analyse spectrale (10) est disposé un collimateur.

11. Dispositif selon la revendication 10, **caractérisé en ce que** le collimateur est réalisé en entrée par un premier condensateur (8) à petite focale et en sortie par un deuxième condensateur (9) à grande focale, le deuxième condensateur (9) étant disposé dans le plan focal du premier condensateur (8).

12. Dispositif selon l'une quelconque des revendications précédentes, **caractérisé en ce que** l'élément d'analyse spectrale (10) est réalisé sous forme de prisme ou de réseau.

13. Dispositif selon l'une quelconque des revendications précédentes, **caractérisé en ce qu'**un groupe d'agents réfrigérants est attribué au dispositif (1), le groupe vaporisant un agent réfrigérant à intervalles de temps fixes et programmables sur la partie principale irradiée.

14. Dispositif selon l'une quelconque des revendications 1 à 12, **caractérisé en ce qu'**un groupe d'agents réfrigérants est attribué au dispositif (1), le groupe se trouvant en liaison opérationnelle avec un capteur de température ou infrarouge et vaporise un agent réfrigérant en cas de dépassement d'une température limite réglable.

15. Dispositif selon la revendication 13 ou 14, **caractérisé en ce que** le point d'ébullition de l'agent réfrigérant se situe entre -80° et -10°C.

16. Dispositif selon l'une quelconque des revendications 1 à 15, **caractérisé en ce que** la source de lumière (3) est réalisée sous forme de lampe à décharge à haute pression ou à basse pression ou sous forme de lampe à incandescence, en particulier sous forme de lampe à halogène.

17. Dispositif selon l'une quelconque des revendications précédentes, **caractérisé en ce que** la source de lumière (3) est recouverte d'une couche de luminophore au niveau du corps en verre.
